Europäisches Patentamt

European Patent Office

Office européen des brevets

(11) Publication number: **0 095 871**
A2

## EUROPEAN PATENT APPLICATION

(21) Application number: **83302895.4**

(22) Date of filing: **20.05.83**

(51) Int. Cl.³: **A 61 K 7/16,** A 61 K 7/24, A 61 K 31/225

(30) Priority: **27.05.82 GB 8215559**

(43) Date of publication of application: **07.12.83**
**Bulletin 83/49**

(84) Designated Contracting States: **AT BE CH DE FR GB IT LI LU NL SE**

(71) Applicant: **RECKITT AND COLMAN PRODUCTS LIMITED, P.O. Box 26 1-17, Burlington Lane, London W4 2RW (GB)**

(72) Inventor: **Pool, Hazel, 211, Lambourn Drive, Allestrey Berby DE3 2UR (GB)**
Inventor: **Cook, Robert Victor, 11, Green Leas, Astonontrent Derbyshire (GB)**

(74) Representative: **Oulton, Richard John et al, H.L. Cottrell & Co. 22, Whitefriargate, Hull North Humberside HU1 2EX. (GB)**

(54) Improvements in or relating to tooth treatment compositions.

(57) This invention relates to tooth treatment compositions and has particular application to desensitising compositions for use on so-called hypersensitive teeth. Tooth sensitivity is exhibited when a sufferer experiences a painful reaction to inter alia pressure, hot, cold, sweet and the like stimulus of a tooth surface and tooth desensitatising compositions for the treatment of tooth hypersensitivity are well known for home use in oral hygene regimens. Such compositions may supplement or replace therapy undertaken by a dentist.
The present invention provides a tooth treatment composition in liquid or paste form including an antihypersensitivity-effective amount of potassium and citrate ions.

- 1 -

"IMPROVEMENTS IN OR RELATING
TO TOOTH TREATMENT COMPOSITIONS"

DESCRIPTION

This invention relates to tooth treatment compositions and has particular application to desensitising
compositions for use on so-called hypersensitive teeth.
Tooth sensitivity is exhibited when a sufferer experiences a painful reaction to inter alia pressure, hot,
cold, sweet and the like stimulus of a tooth surface and
tooth desensitising compositions for the treatment of
tooth hypersensitivity are well known for home use in
oral hygiene regimens.   Such compositions may supplement
or replace thereapy undertaken by a dentist.

Such prior art compositions may be in the form of:-
mouthwashes comprising aqueous solutions or dispersions;
solutions, dispersions or gels for painting the affected
area of the tooth; powders or pastes for application to

0095871

the affected and neighbouring areas; toothpastes or dentifrices containing the treatment material in suspensions or solution for cleaning teeth; and chewable tablets in which the treatment material is contained.

Examples of treatment materials so far proposed for reducing hypersensitivity fall into two classes. One class is of protein precipitants, for example, formalin, silver nitrate, strontium chloride and phenol. The second class, hypothetically, are tubule occluding agents, for example certain fluorides, calcium hydroxide, calcium phosphate, nitrates of alkali metals especially potassium nitrate, strontium chloride also silver nitrate and formalin.

By way of example the United States of America Patent No. 3863006 advocated alkali metal nitrates, especially potassium nitrate, for use in aqueous solution or toothpaste or dentifrice in a method of treating hypersensitive teeth.

Further, in United States of America Patent No. 4011309 citric acid and trisodium citrate dihydrate are advocated for use together in an aqueous gel containing as essential ingredient certain water-soluble, non-ionic polyol surfactants in the treatment of hypersensitive teeth.

However, a feature of the prior art formulations is that the desensitizing component has an unpleasant salt flavour, which is difficult to mask, rendering compositions containing them more or less unpleasant to use.

It is an object of the present invention to provide a composition for efficacious treatment of tooth hypersensitivity, but one free from the above-stated disadvantage.

Accordingly, the present invention provides a tooth treatment composition in liquid or paste form including, to counter tooth hypersensitivity, potassium and citrate ions in antihypersensitivity-effective amount.

The potassium citrate may be furnished by a nontoxic water-soluble potassium salt and a water-soluble citrate salt.

The composition may include a potassium salt and a citrate which furnish appropriate ions characteristic of a potassium citrate, the counterions forming an innocuous product.

The term "citrate" includes citric acid and/or water-soluble salts thereof.

The potassium citrate may have a non-stoichiometric composition.

Preferably the composition includes a carrier suitable for use in the buccal cavity.

When a potassium salt other than a potassium citrate is used said salt furnishes the necessary potassium ions in the buccal cavity.

Similarly, when the citrate is present the necessary citrate ions are furnished in the buccal cavity.

Preferably the composition has, or generates in use, a pH of from 5 to 9.5, more preferably the pH does not exceed 8.7 and for some compositions the pH is between 5 and 6.5.

In the case that the composition is a dentifrice, the pH thereof may by custom have a value in the range pH 4.5 to pH 10.5. It is worth remarking that a pH value so high as pH 10.5 can be at least awkward to achieve in the presence of very large amounts of citrate and/or citric acid which act as pH-buffering compositions.

The composition may be in the form of an aqueous or alcoholic or glycerol based composition in the form of a mouthwash, a solution for direct application to a tooth surface as by painting, a toothpaste or dentifrice.

The invention also envisages non-aqueous powders or tablets, for the preparation of aqueous compositions within the scope of this invention, and which may be used in admixture with water alone or other suitable media, including aqueous media for example one containing alcohol or glycerol or the like.

The amount of potassium and citrate ions furnished is preferably selected in accordance with the precise nature of the composition providing them, that is whether the composition be a mouthwash, a painting fluid, a toothpaste or dentifrice, or a powder/tablet for preparing aqueous solutions.

Preferably the composition includes a compound of general formula:

$$K_nH_{3-n}C_6H_5O_7$$

wherein n is preferably an integer, = 1, 2 or 3.

Alternatively a chemical equivalent in ionic terms of the compounds so described is contained in the composition.

Alternatively, the value of n is fractional in which case the gram-ion ratio of potassium ions to citrate ions in the composition is at least 1:10, preferably 1:7, more preferably at least 2:1 and more particularly at least 3:1.

Preferably the composition includes up to 40% by weight of a potassium citrate or the equivalent thereof.

When the commonly occurring tripotassium citrate, $K_3C_6H_5O_7 \cdot H_2O$ of commerce is used and which is a salt of a strong base with a relatively weak acid, as indicated by the third dissociation constant $6.4 \times 10^{-6}$ at 18°C, of citric acid, an amount of free citric acid may be admixed to bring the pH down to near neutral. For example, the preferred pH of an acidic toothpaste or dentifrice is 5.0 to 6.5.

When the monopotassium salt, $KH_2C_6H_5O_7$ is used and which is a salt of a strong base and a strong organic acid as indicated by the first dissociation constant, $7.1 \times 10^{-4}$ of citric acid, a relatively high concentration of citric acid may not be required for pH adjustment.

Dipotassium citrate, $K_2HC_6H_5O_7$ may be employed. It may be noted that the second dissociation constant of citric acid is $1.68 \times 10^{-5}$ at 18°C, an acid of strength intermediate the strength of the other two. As a consequence the concentration of citric acid required as a buffer will be an intermediate value.

Thus, it may be seen that the concentrations of potassium and citrate ions will vary in accordance with the nature of the composition and, additionally, the concentrations of citric acid and citrate will vary with the potassium ion concentration in accordance with buffering requirements.

Preferably, the potassium citrate used is dipotassium citrate. Free citric acid may be employed in conjunction therewith if desired for the purposes defined above.

When the source of potassium ions is other than the above dipotassium salt, free citric acid is preferably also present as the source of citrate ion but citrate salts may be used.

Conveniently, the relative amounts of potassium and citrate ions present are the stiochiometric proportions thereof in dipotassium citrate.

In one embodiment in accordance with the invention the citrate may be in excess of the stoichiometric amount.

The amount of potassium and citrate ions present may be at least commensurate with the stoichiometric proportions thereof in monopotassium citrate.

The salts and acids may be employed in their hydrated forms, due allowance being made for the weight extent of hydration.

When the composition is a toothpaste or dentifrice, the amount of $K_nH_{(3-n)}C_6H_5O_7$ or the equivalent thereof present may be up to 10% by weight of the composition as a whole.

For example, a toothpaste or dentifrice may contain the equivalent of 0.3% to 9% by weight of a potassium citrate, especially 0.6% to 3% may be employed.

When the composition is a mouthwash, paintable composition or composition for the preparation thereof, the amount of $K_nH_{3-n}C_6H_5O_7$ or the equivalent thereof may exceed the preferred amount.

- 8 -

0095871

Where a treatment composition is designed for frequent repeat dose usage in an ordinary oral hygiene regimen, such as in a toothpaste or dentifrice or a mouthwash, the equivalent concentration of potassium citrate may be reduced.

In toothpastes or dentifrices in accordance with the present invention abradants polishing aids or other materials that adsorb, precipitate or otherwise sequester the potassium and/or citrate ions must be allowed for.

Polymeric materials such as those based upon acrylates, vinyl chloride, olefines and mixtures thereof in particulate or bead form are preferred as abradants or polishing aids and offer the advantage of relatively controllable abrasivity.

Typically, toothpastes or dentifrices in accordance with the present invention will contain the usual sorts of components types and they may be anhydrous, such as those based on glycerol only; or they may be water-based. The latter usually contain humectants, foaming agents, binding or suspending agents, abradants, preservatives, flavourants and water. The salts and acids proposed by the present invention are most easily incorporated in the aqueous or glycerol phase.

As humectant glycerol, sorbitol, propylene glycol, and the like may be employed.

Conveniently, the foaming agent is a surfactant or surfactant blend of amphoteric, anionic or nonionic nature for example, amine oxides, sodium lauryl sulphate, the TWEENS.

The binding or suspending agents conveniently include gums such as xanthan gum, and thickeners such as bentonite, carboxymethylcellulose and derivatives, laponite and magnesium aluminium silicate.

In addition to the above-mentioned organic abradants the usual inorganic abradants, including aluminosilicates, chalks and phosphate may be employed having regard to the above restrictions regarding sequestation.

Other treatment materials, for example for halitosis, anti plaque and plaque removal, may be incorporated into a toothpaste or dentifrice. Additionally, compounds furnishing fluoride such as sodium gluoride, stannous fluoride, amine fluorides and sodium monofluorophosphate may be used. Such additaments are intended to provide all round protection in the one composition.

In the above embodiments the ratio of citrate ions to potassium ions is preferably at least 0.3:1.

The invention is further illustrated with reference to the following non-limitative Examples in which parts and percentages are by weight of the total composition.

Example 1

An alcoholic mouthwash was prepared by blending the following components

|  | %age w/w |
|---|---|
| Ethanol | 15.0 |
| Water | 71.58 |
| Sorbitol | 10.0 |
| Sodium benzoate | 0.1 |
| Benzoic acid | 0.1 |
| Colourant | 0.4 |
| Polyoxyethylene (20mol) sorbitan monoisostearate | 0.22 |
| Flavourant | 0.1 |
| Dipotassium citrate | 2.0 |
| Citric acid | 0.5 |

As preliminary steps the benzoic acid was dissolved in alcohol, and the sodium benzoate was dissolved in water.

The product was a brilliant, clear liquid.

Example 2

A non-alcoholic mouthwash containing glycerol was prepared by blending the following components.

|  | %age w/w |
|---|---|
| Water | 86.68 |
| Glycerin | 10.0 |
| Sodium benzoate | 0.1 |
| Colourant | 0.4 |
| Flavourant | 0.1 |
| Polyoxyethyleneoxide (20mol) sorbitan monoisostearate | 0.22 |

%age w/w

Dipotassium citrate                                          2.0

Citric acid                                                  0.5 .

The product was initially hazy but after standing for 48 hour it assumed a clear brilliance.

EXAMPLE 3

A dentifrice containing an inorganic abradant was prepared from 1.67 parts dipotassium citrate, 0.5 parts citric acid BP, 0.15 parts sodium saccharin B.P., 0.1 parts p-hydroxy methyl benzoate which was dissolved in the major part of the water mixed with 15 parts sorbitol and 5 parts glycerol with stirring at room temperature under reduced pressure in a vacuum mixer. To this mixture was added 20 parts sodium aluminosilicate with stirring. 0.75 parts of a xanthan gum was sprinkled over the surface and gently stirred in until the mixture was thickened and homogeneous. 1.6 parts sodium carboxymethyl cellulose in 5 parts glycerine was next mixed and the whole stirred until homogeneous. 1.2 parts of flavourant and 1.35 parts sodium lauryl sulphate dispersed in 10% of the total water used was then added with mixing until homogeneous. The product was a stable toothpaste or dentifrice.

In a modification 0.8 parts sodium monofluorophosphate was introduced with the powders in the initial mixing step to provide all round protection for the teeth when the toothpaste or dentifrice is regularly used.

EXAMPLE 4

A dentifrice containing an acrylic abradant (replacing the sodium aluminosilicate of Example 3) was prepared using the following ingredients in the above method of Example 3

|  | Parts by weight |
|---|---|
| Sodium carboxymethyl cellulose | 1.5 |
| Dipotassium citrate | 2.0 |
| Glycerin B.P. | 10.0 |
| Sorbitol B.P. | 15.0 |
| p-hydroxylmethyl benzoate | 0.1 |
| Sodium saccharin B.P. | 0.1 |
| Sodium lauryl sulphate | 1.35 |
| Acrylic polymer JMP67 | 15.0 |
| Flavour and colourant | q.s |
| Water | ad 100.0 |

The product was a stable, i.e. non-separating paste with a pleasant mouthfeel.

Sodium monofluorophosphate was introduced into the composition at the expense of the total water content at a level of 0.8% to provide all-round protection for the teeth when used regularly.

EXAMPLE 5 .

A dentifrice containing a wholly inorganic abradant was prepared from the following ingredients:-

|  | %age w/w |
|---|---|
| Tripotassium citrate | 1.67 |
| Citric acid | 0.50 |

- 13 -

0095871

|  | %age w/w |
|---|---|
| Glycerine B.P. | 10.00 |
| Sodium carboxymethylcellulose | 1.60 |
| Sorbitol B.P. | 15.00 |
| Sodium monofluorophosphate | 0.80 |
| Sodium saccharin B.P. | 0.15 |
| p-hydroxymethyl benzoate | 0.10 |
| Titanium dioxide B.P. | 0.10 |
| Xanthan gum | 0.75 |
| Sodium lauryl sulphate | 1.35 |
| Sodium aluminosilicate | 15.00 |
| Synthetic precipitated amorphous silica | 5.00 |
| Flavourant | 1.20 |
| Water | ad 100.0 |

The xanthan gum was dispersed in part of the water and homogenised. To this dispersion the following ingredients were added with stirring under 800 mbar vacuum: potassium citrate, citric acid, half the glycerine, sorbitol, sodium monofluorophosphate, sodium saccharin, methyl benzoate, pigment and blended abradant. A dispersion of the sodium carboxymethylcellulose in half the glycerine was then added followed by a solution of the sodium lauryl sulphate in about one tenth of the water. The flavourant was then added and whole stirred to a uniform air-free mixture of pH 8.45 prior to filling into tubes with avoidance of the introduction of air bubbles.

The toothpaste when tested by volunteers was found to have an agreeable texture, flavour and after taste. It was generally considered to provide ample foaming and cleansing efficacy. Further, both volunteers and the dentists conducting the tests on hypersensitivity found the composition to be at least as efficacious in reducing the effects of hypersensitivity.

The compositions proposed by the present invention may be self administered or applied by any other persons not necessarily qualified as a dentist or oral hygiene practitioner.

Furthermore, the treatment materials of the present invention, being of a bland nature, enable the incorporation of a miscellany of other treatment materials to be incorporated in the one composition.

CLAIMS:

1.    A tooth treatment composition in liquid or paste form including, to counter tooth hypersensitivity, potassium and citrate ions in antihypersensitivity-effective amount.

2.    A composition as claimed in claim 1 in which the gram-ion ratio of potassium to citrate ion present is at least 1:10.

3.    A composition as claimed in claim 2 in which the gram-ion ratio of potassium to citrate ion present is in the range of at least 1:1.

4.    A composition as claimed in claim 2 in which the potassium and citrate ions are present in stoichiometric amounts in a compound of formula $K_nH_{3-n}C_6H_5O_7$, wherein n = 1, 2 or 3.

5.    A composition as claimed in any preceding claim in which the potassium and citrate ions present, expressed as $K_nH_{3-n}C_6H_5O_7$, wherein n = 1, 2 or 3, amount to up to 40% by weight of the total composition.

6.    A composition as claimed in claim 5 in which the potassium and citrate ions amount up to 10% by weight of the total composition.

7.    A composition as claimed in claim 5 in which the amount of potassium and citrate ions is from 0.3% to 9% by weight of the total composition.

8.    A composition as claimed in any preceding claim which is a dentifrice.

9.    A composition as claimed in claim 8 including an abradant.

10.    A composition as claimed in claim 9 in which the abradant includes a polymeric material of the group consisting of acrylics, polyvinyl chloride and polyolefines in particulate or bead form.